# EUROPÄISCHE PATENTSCHRIFT

(11) **EP 0 691 422 B1**
(45) Veröffentlichungstag und Bekanntmachung des Hinweises auf die Patenterteilung: **03.11.1999**
(21) Anmeldenummer: 95109588.4
(22) Anmeldetag: 21.06.1995
(51) Int. Cl.: C25B 3/10, C07C 271/22, C07C 227/16, C07C 229/26, C07C 233/01

(54) **Verfahren zur Herstellung substituierter Diaminodicarbonsäurederivate**
Process for the preparation of substituted diaminodicarboxylic acid derivatives
Procédé de préparation de dérivés d'acide diaminodicarboxylique substituées

(30) Priorität: 08.07.1994 AT 134894
(43) Veröffentlichungstag der Anmeldung: 10.01.1996
(73) Patentinhaber: Nycomed Austria GmbH, 4020 Linz (AT)
(72) Erfinder: Hiebl, Johann, Mag. Dr., A-4030 Linz-Pichling (AT); Rovenszky, Franz, Dipl. Ing. Dr., A-4020 Linz (AT)
(74) Vertreter: Matthews, Derek Peter

(56) Entgegenhaltungen:
- JOURNAL ORGANIC CHEMISTRY, Bd. 45, Nr. 15, 1980 Seiten 3078-3080, R.F. NUTT 'USEFUL INTERMEDIATES FOR SYNTHESIS OF DICARBA ANALOGUES OF CYSTINE PEPTIDES'

## Beschreibung

Die Erfindung betrifft ein neues Verfahren zur Herstellung substituierter Diaminodicarbon-säurederivate in hoher Ausbeute mittels Kolbe-Synthese.

Substituierte Diaminodicarbonsäurederivate sind wertvolle Zwischenprodukte in der Synthese von Peptiden.

In der bislang effektivsten Synthese wird beispielsweise N-t-butyloxycarbonylglutaminsäure-α-benzylester einer Kolbe-Elektrolyse unterzogen. Bei dieser im Basischen ablaufenden Reaktion wird das Anion der geschützten Aminosäure zunächst oxidiert und unter CO₂-Abspaltung in ein Radikal-Intermediate umgewandelt. Dieses Radikal kann nun mit einem Solvens-Proton reagieren, oder seinerseits unter Ausbildung einer Doppelbindung ein Wasserstoffatom abgeben, während Reaktion mit einem zweiten Radikal zum gewünschten Diaminodicarbonsäurederivat führt.
Der entscheidende Nachteil dieser Methode ist eine Nebenreaktion, bei der das Reaktionsprodukt mit dem Alkohol des Lösungsmittels reagieren und so teilweise oder vollständig umgeestert werden kann. Dieser Prozeß reduziert natürlich die Ausbeute an reinem, isolierten Produkt beträchtlich, die maximal 20% der Theorie beträgt, auch ist das entstehende Produktgemisch nur besonders schwierig und aufwendig zu reinigen.

Auf J. Org. Chem. 45, ss 3078 - 3080 (1980) wird auch hingewiesen.

Überraschenderweise konnte ein Verfahren zur Synthese von Diaminodicarbonsäurederivaten gefunden werden, das die Bildung von Umesterungsprodukten vermeidet.

Gegenstand der Erfindung ist daher ein Verfahren zur Herstellung substituierter Diaminodicarbonsäurederivate der Formel 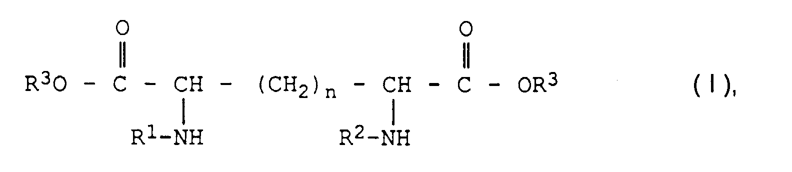 in der R¹ und R² jeweils unabhängig voneinander einen gegebenenfalls halogenierten geradkettigen, verzweigten oder zyklischen Alkylrest mit 1-10 C-Atomen oder einen Rest 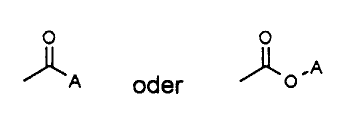 bedeutet, wobei A einen gegebenfalls halogenierten geradkettigen, verzweigten oder zyklischen Alkylrest mit 1-10 C-Atomen oder einen gegebenenfalls ein- oder mehrfach oder gemischt durch Halogen, -NO₂, Alkoxy oder -CN substituierten Benzylrest oder 9-Fluorenylmethyl und R³ einen geradkettigen oder verzweigten Alkylrest mit 1-4 C-Atomen bedeuten, wobei die Chiralitätszentren in den Molekülen durch die verwendeten Edukte bestimmt werden und beide L oder beide D oder D,L bzw. L,D konfiguriert sein können und n eine ganze Zahl von 2-8 bedeutet,
durch gegebenenfalls gemischte Kolbe-Synthese, das dadurch gekennzeichnet ist, daß ein geschütztes Aminosäurederivat der Formel 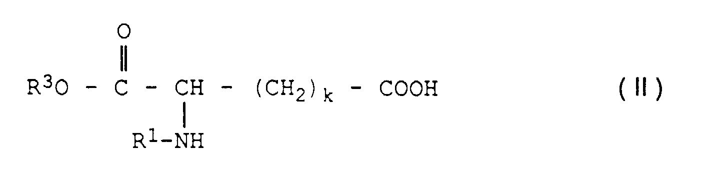 in der R¹ und R³ die oben genannte Bedeutung haben und k eine ganze Zahl bedeutet, mit einem geschützten Aminosäurederivat der Formel 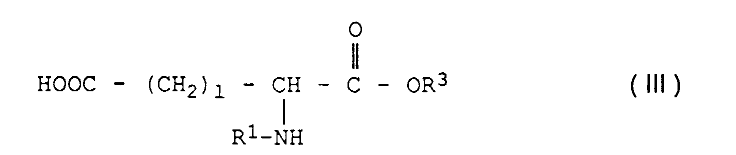 in der R¹ und R³ die oben genannte Bedeutung haben und l eine ganze Zahl bedeutet, wobei k und l zusammen die Zahl n ergeben,
in einem Lösungsmittel R⁴OH, wobei R⁴ die Bedeutung von R³ hat, oder einem heterozyklischen oder aliphatischen, mindestens ein Heteroatom enthaltenden Lösungsmittel oder Mischungen solcher Lösungsmittel gelöst wird und einer Elektrolyse an Platinnetzelektroden unterworfen und das Produkt gegebenenfalls mit LiOH hydrolisiert wird.

Der Vorteil dieses Verfahrens besteht einerseits in der hohen Ausbeute an reinem, isolierten Produkt von mindestens 34% der Theorie, andererseits kann durch den geringeren Anteil an Nebenprodukten der Reinigungsaufwand erheblich reduziert werden. Daher ist dieses Verfahren geeignet, die Herstellungskosten von Folgeprodukten, wie Peptiden und nicht natürlich vorkommende Aminosäuren enthaltenden Verbindungen, erheblich zu reduzieren.

In den Formeln I, II und III bedeuten R¹ und R² jeweils einen gegebenenfalls halogenierten geradkettigen, verzweigten oder zyklischen Alkylrest mit 1-10 C-Atomen, beispielseise Methyl-, Ethyl-, n-Propyl-, i-Propyl-, n-Butyl-, i-Butyl, t-Butyl, Pentyl- oder Hexylrest, der gegebenenfalls ein- oder mehrfach halogeniert sein kann.

Weiters können R¹ und R² einen Rest 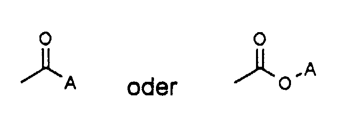 bedeuten, wobei A 9-Fluorenylmethyl- oder einen gegebenenfalls ein- oder mehrfach oder gemischt durch Halogen, NO₂, Alkoxy oder -CN substituierten Benzylrest bedeutet, beispielsweise einen Brombenzyl-, Dibrombenzyl-, Chlorbenzyl-, Dichlorbenzyl-, Nitrobenzyl-, Methoxybenzyl oder Cyanobenzylrest.

Vorzugsweise bedeuten R¹ und R² jeweils einen Rest 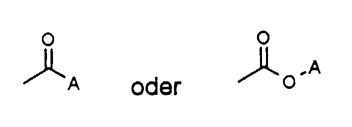 wobei A einen 9-Fluorenylmethylrest, einen gegebenenfalls substituierten Benzylrest oder einen geradkettigen oder verzweigten Alkylrest mit 1-4 C-Atomen bedeutet.

Der Rest R³ bedeutet einen geradkettigen oder verzweigten Alkylrest mit 1-4 C-Atomen, beispielsweise Methyl-, Ethyl-, n-Propyl-, i-Propyl-, n-Butyl-, i-Butyl-, t-Butyl-, vorzugsweise Methyl, Ethyl oder i-Propyl.

Die Chiralitätszentren der Dicarbonsäuren werden durch die Wahl der verwendeten Edukte bestimmt. Sie können entweder beide D oder beide L oder D,L bzw. L,D, beispielsweise N'-R¹-N''-R²-2,7-D,L-2,7-diaminosuberinsäure-di-R³-ester bei Verwendung von N-R¹-D-glutaminsäure-R³-ester und N-R²-L-glutaminsäure-R³-ester, konfiguriert sein.

Die Aminosäurederivate der Formeln II und III werden in einem Lösungsmittel R⁴OH oder einem heterozyklischen Lösungsmittel wie beispielsweise Pyridin oder Dimethylformamid oder Acetonitril oder Mischungen solcher Lösungsmittel gelöst, wobei R⁴ die Bedeutung von R³ hat, also beispielsweise Methyl-, Ethyl-, n-Propyl-, i-Propyl-, n-Butyl-, i-Butyl- oder t-Butyl-, vorzugsweise Methyl, Ethyl oder i-Propyl bedeutet..

In der Elektrolysezelle wird der Lösung eine Base zugefügt, beispielsweise Alkalimetall in R⁴OH, etwa Natriummethoxid in Methanol, oder Kaliumethoxid in Ethanol.
Anschließend wird an Platinnetzelektroden unter Kühlen elektrolysiert, wobei die Temperatur vorzugsweise auf 18-25 °C gehalten wird. Die Stromstärke bei der Elektrolyse beträgt etwa 5-15 A bei 60-120 V angelegter Spannung und ist im übrigen abhängig von der Geometrie der verwendeten Elektrode.
Der Elektrolysevorgang wird beendet, sobald kein Edukt in der Elektrolyselösung mehr festgestellt werden kann.
Die Elektrolyselösung wird anschließend gegebenenfalls unter niedrigem Druck eingeengt, der Rückstand in einem geeigneten Lösungsmittel, beispielsweise Ethylacetat aufgenommen und diese Lösung nacheinander mit verdünnter Säure, beispielsweise verdünnter Salzsäure, einer gesättigten Salzlösung, beispielsweise einer gesättigten Natriumhydrogencarbonatlösung, und gesättigter Natriumchloridlösung gewaschen.
Die Lösung wird anschließend mit einem geeigneten Trocknungsmittel, beispielsweise Natriumsulfat oder Magnesiumsulfat, getrocknet, filtriert und erneut, gegebenenfalls unter niedrigem Druck, eingeengt.

Der Rückstand wird chromatographisch gereinigt, beispielsweise über Silicagel, was wegen des geringen Anteils an Nebenprodukten mit vergleichsweise geringem Aufwand möglich ist. Gegenüber einem früheren Verfahren, bei dem R³ und R⁴ nicht identisch waren, verläuft die Reaktion in einer Ausbeute von bis zu 35% d.Th. im Gegensatz zu früheren 10-15%.

### Beispiel 1:

47,40 g (181 mmol) N-t-Butyloxycarbonylglutaminsäure-α-t-methylester wurden in 240 ml MeOH und 80 ml Pyridin durch Umschwenken gelöst. Die Reaktionslösung wurde in die Elektrolysezelle mit zylinderförmig angeordneten Platinnetzelektroden transferiert. Es wurde mit MeOH nachgespült und die Elektrolysezelle soweit mit MeOH aufgefüllt, daß beide Elektroden vollständig eintauchten.
Nun wurden 0,8 ml NaOCH₃ (30% in MeOH) zugegeben, und die Reaktionslösung auf 15°C gekühlt. Die Reaktionstemperatur wurde durch Temperaturregelung bzw. durch Regelung der Stromstärke bzw. -spannung (5-15 A, 60-120 V) zwischen +18°C und +24°C gehalten.
Der Reaktionsverlauf wurde mittels DC kontrolliert.
Nach vollständiger Reaktion wurde die Reaktionslösung bei 40 °C einrotiert.

Der Rückstand der Kolbe-Synthese wurde in 250 ml Ethylacetat gelöst, zuerst mit verdünnter HCl-Lösung (75 ml HCl conc. mit H₂O auf 200 ml aufgefüllt), dann mit 200 ml NaHCO₃ ges. und zuletzt mit je 200 ml NaCl ges. bis zur Neutralität der wäßrigen Phase gewaschen.

Die organische Phase wurde mit Na₂SO₄ getrocknet, abfiltriert, und eingedampft. Eindampfrückstand: 37,93 g.

Der Eindampfrückstand wurde über Kieselgel filtriert und anschließend mittels Säulenchromatographie aufgetrennt.

Die Produkt enthaltenden Fraktionen wurden konzentriert und aus dem öligen Rückstand (24,08 g) wurden aus 100 ml Petrolether: Cyclohexan = 3: 1 13,69 g farblose Kristalle erhalten.

Ausbeute: 13,69 g des reinen Bis-N',N''-Benzyloxycarbonyl-2,7-diaminosuberinsäuredimethylester (35% d.Th.), Fp.65-68 C.
¹³C(CDCl₃, 100MHz): 24.86(2CH₂), 28.30((**C**H₃)₃C), 32.53(2CH₂), 52.17(2 OMe), 53.29, (2CH), 79.87(2(CH₃)₃**C**), 155.31 (2carbamat-CO), 173.20(2ester-CO).

### Beispiel 2:

1,5 g (1,16 mmol) Bis-N',N''-Benzyloxycarbonyl-2,7-diaminosuberinsäuredimethylester wurden in einer Lösung aus 1,5 ml Wasser und 3 ml MeOH gelöst. Zu dieser Lösung wurden 1,45 ml (2,90 mmol, 1,25 Äquivalente) einer 2N LiOH-Lösung in Wasser gegeben. Durch vorsichtige Zugabe von etwa 1 ml MeOH entstand aus der leicht trüben eine klare Lösung. Die Reaktionslösung wurde bei Raumtemperatur gerührt und anschließend am Rotavapor auf 3 ml konzentriert und der pH-Wert der Lösung mit 5% KHSO₄-Lösung auf 2-3 gestellt. Die saure Lösung wurde mit Chloroform extrahiert, die organische Phase mit Na₂SO₄ getrocknet, filtriert und konzentriert. Es wurde 0,555 g an Rohprodukt erhalten, das aus Acetonitril umkristallisiert wurde.

Ausbeute: 0,324 g (80%). Untersuchung mit chiraler Kapillarelektrophoese ergibt, daß keine Racemisierung nachweisbar ist.

Analog zu den Beispielen 1 und 2 wurden folgende Verbindungen synthetisiert:
Di-Boc-D,D-SUB-di-OMe
Ausbeute: 1.15g, (35% d. Th.), Schmp.:42-47°C.
¹H NMR (400 MHz, CDCl₃) δ 4.99(s, 2 H, 2 NH), 4.27(s, 2 H, 2 CH), 3.73(s, 6 H, 2 OMe), 1.76 und 1.60 (m, 2 H), 1.44(s, 18 H), 1.35(m, 4 H).
¹³C NMR (100 MHz, CDCl₃) δ 173.2, 155.3, 79.9, 53.3, 52.2, 32.5, 28.3, 24.9.

### Di-Boc-D,D-SUB

Ausbeute: 0.37g (79% d. Th.). Schmp.: 152-154°C.
¹H NMR (400 MHz, MeOH-d₃) δ 4.12(s, 2 H, 2 CH), 1.82 und 1.70(m, 2 H), 1.48(s, 18 H, 2 C(CH₃)₃), 1.45(m, 4 H, 2 CH₂).
¹³C NMR (100 MHz, DMSO-d₆) δ 176.5, 158.1, 80.8, 55.1, 33.0, 29.0, 26.8.

### Di-BOC-L,L-SUB-di-OEt

Ausbeute: 7.29g HN-60204, (33.6% d. Th.), leicht gelbliches Öl.
¹H NMR (400 MHz, CDCl₃) δ 5.00(br s,1 H, NH), 4.26(br s, 1 H, CH), 4.19(q, 2 H, OC*H*₂CH₃), 2.46(m, 2 H), 1.78 und 1.60(m, 2 H), 1.44(s, 18 H), 1.35(m, 4 H), 1.28(t, 3 H, OCH₂C*H*_{*3*}).
¹³C NMR (100 MHz, CDCl₃) δ 172.4, 155.0, 79.4, 60.9, 53.1, 32.3, 28.0, 24.6, 143.9.

### Di-BOC-L,L-SUB

Ausbeute: 3.7g (84%), Schmp.: 150-153°C.

| C,H,N-Analyse | C | H | N |
|---|---|---|---|
| Berechnet | 53.4 | 7.97 | 6.93 |
| Gefunden | 53.2 | 8.1 | 6.8 |

Die oben verwendeten Abkürzungen bedeuten:
- Boc:: t-Butyloxycarbonyl
- SUB:: 2,7-Diaminosuberinsäure
- Me:: Methyl
- Et:: Ethyl

## Patentansprüche

1. Verfahren zur Herstellung substituierter Diaminodicarbonsäurederivate der Formel 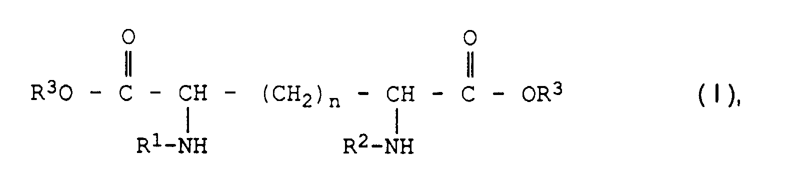 in der R¹ und R² jeweils unabhängig voneinander einen gegebenenfalls halogenierten geradkettigen, verzweigten oder zyklischen Alkylrest mit 1-10 C-Atomen oder einen Rest 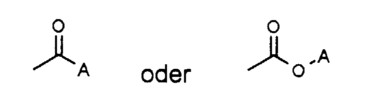 bedeutet, wobei A einen gegebenfalls halogenierten geradkettigen, verzweigten oder zyklischen Alkylrest mit 1-10 C-Atomen oder einen gegebenenfalls ein- oder mehrfach oder gemischt durch Halogen, -NO₂, Alkoxy oder -CN substituierten Benzylrest oder 9-Fluorenylmethyl
und R³ einen geradkettigen oder verzweigten Alkylrest mit 1-4 C-Atomen bedeuten, wobei die Chiralitätszentren in den Molekülen durch die verwendeten Edukte bestimmt werden und beide L oder beide D oder D,L bzw. L,D konfiguriert sein können und n eine ganze Zahl von 2-8 bedeutet, durch gegebenenfalls gemischte Kolbe-Synthese, das dadurch gekennzeichnet ist, daß ein geschütztes Aminosäurederivat der Formel 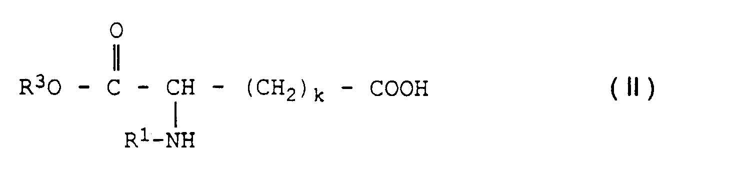 in der R¹ und R³ die oben genannte Bedeutung haben und k eine ganze Zahl bedeutet, mit einem geschützten Aminosäurederivat der Formel 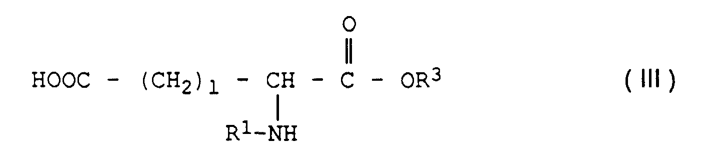 in der R¹ und R³ die oben genannte Bedeutung haben und l eine ganze Zahl bedeutet, wobei k und l zusammen die Zahl n ergeben,
in einem Lösungsmittel R⁴OH, wobei R⁴ die Bedeutung von R³ hat, oder einem heterozyklischen oder aliphatischen, mindestens ein Heteroatom enthaltenden Lösungsmittel, oder Mischungen solcher Lösungsmittel gelöst wird und einer Elektrolyse an Platinnetzelektroden unterworfen und das Produkt gegebenenfalls mit LiOH hydrolisiert wird.

2. Verfahren zur Herstellung solcher substituierter Diaminodicarbon-säurederivate der Formel I nach Anspruch 1, wonach R¹ und R² jeweils einen Rest 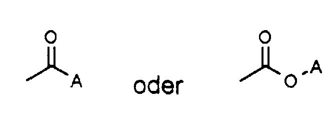 bedeuten, wobei A einen gegebenenfalls halogenierten geradkettigen, verzweigten oder zyklischen Alkylrest mit 1-10 C-Atomen oder einen gegebenenfalls substituierten Benzylrest oder 9-Fluorenylmethyl und R³ einen geradkettigen oder verzweigten Alkylrest mit 1-4 C-Atomen bedeuten, wobei die Reaktion in einem Lösungsmittelgemisch aus R⁴OH, wobei R³ und R⁴ identisch sind, und Pyridin, Dimethylformamid oder Acetonitril stattfindet.

## Claims

1. Process for the preparation of substituted diaminodicarboxylic acid derivatives of the formula 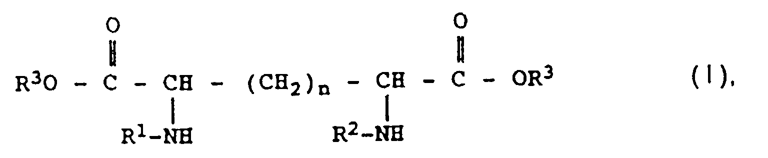 in which R¹ and R² in each case independently of one another are an optionally halogenated straight-chain, branched or cyclic alkyl radical having 1-10 C atoms or a radical 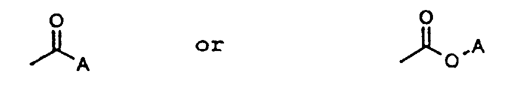 wherein A is an optionally halogenated straight-chain, branched or cyclic alkyl radical having 1-10 C atoms or a benzyl radical which is optionally mono- or polysubstituted by identical or different halogen, -NO₂, alkoxy or -CN substituents, or is 9-fluorenylmethyl
and R³ is a straight-chain or branched alkyl radical having 1-4 C atoms, the chirality centres in the molecules being determined by the starting materials used and it being possible for both to be in the L or both to be in the D or D,L or L,D configuration, and n is an integer from 2 to 8,
by optionally mixed Kolbe synthesis, which is characterized in that a protected amino acid derivative of the formula 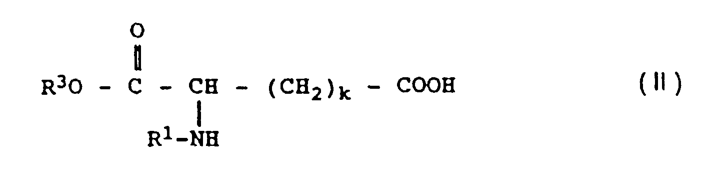 in which R¹ and R³ have the abovementioned meaning and k is an integer, is dissolved with a protected amino acid derivative of the formula 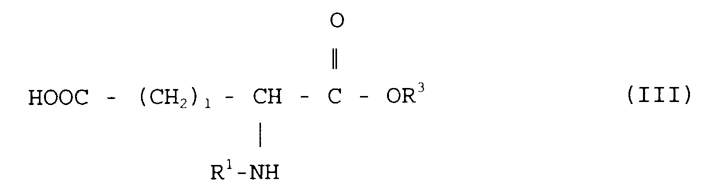 in which R¹ and R³ have the abovementioned meaning and l is an integer, wherein k and l together give the number n,
in a solvent R⁴OH, wherein R⁴ has the meaning of R³, or a heterocyclic or aliphatic solvent containing at least one heteroatom or mixtures of such solvents, the solution is subjected to electrolysis on platinum gauze electrodes and, if appropriate, the product is hydrolysed with LiOH.

2. Process for the preparation of those substituted diaminodicarboxylic acid derivatives of the formula I according to claim 1, in which R¹ and R² are each a radical 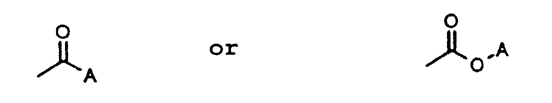 wherein A is an optionally halogenated straight-chain, branched or cyclic alkyl radical having 1-10 C-atoms or an optionally substituted benzyl radical or 9-fluorenylmethyl and R³ is a straight-chain or branched alkyl radical having 1-4 C atoms, the reaction taking place in a solvent mixture of R⁴OH, wherein R³ and R⁴ are identical, and pyridine, dimethylformamide or acetonitrile.

## Revendications

1. Procédé de préparation de dérivés d'acides diaminocarboxyliques substitués de formule : 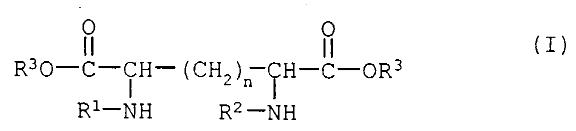 dans laquelle R¹ et R² représentent indépendamment l'un de l'autre un résidu alkyle linéaire,
ramifié ou cyclique, le cas échéant halogéné, ayant de 1 à 10 atomes de carbone, ou bien un résidu 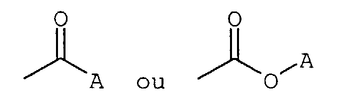 dans lequel A est un résidu alkyle linéaire,
ramifié ou cyclique, le cas échéant halogéné, ayant de 1 à 10 atomes de carbone, ou bien un résidu benzyle le cas échéant mono - ou plurisubstitué ou substitué de manière mixte par un groupe halogène, -NO₂, alcoxy ou -CN, ou bien un groupe 9-fluorénylméthyle ; R³ est un résidu alkyle à chaîne droite ou ramifiée ayant de 1 à 4 atomes de carbone, les centres de chiralité des molécules étant déterminés par les éduits utilisés et pouvant avoir une configuration soit L l'un et l'autre, soit D l'un et l'autre, soit D,L, soit L,D ; et n est un nombre entier de 2 à 8 ; par une synthèse de Kolbe le cas échéant mixte, procédé caractérisé en ce que l'on dissout un dérivé d'aminoacide protégé de formule : 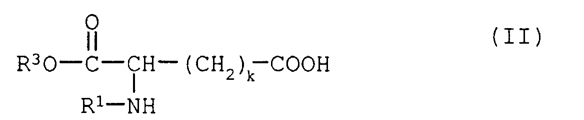 dans laquelle R¹ et R³ ont la signification indiquée ci-dessus et k est un nombre entier, avec un dérivé d'aminoacide protégé de formule : 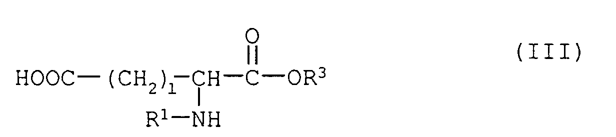 dans laquelle R¹ et R³ ont la signification indiquée ci-dessus et 1 est un nombre entier, la somme de k et 1 étant égale à n, dans un solvant R⁴OH, où R⁴ à la même signification que R³, ou bien dans un solvant hétérocyclique ou aliphatique contenant au moins un hétéroatome ou dans un mélange de solvants de ce type, et on le soumet à une électrolyse au moyen d'électrodes à mailles en platine et, le cas échéant, on hydrolyse le produit avec du LiOH.

2. Procédé de préparation de dérivés d'acides diaminocarboxyliques substitués de la formule I selon la revendication 1, dans lesquels R¹ et R² représentent l'un et l'autre un résidu 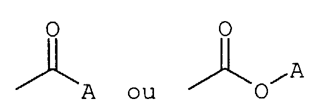 dans lequel A est un résidu alkyle linéaire,
ramifiée ou cyclique, le cas échéant halogéné, ayant de 1 à 10 atomes de carbone, ou bien un résidu benzyle le cas échéant substitué, ou bien un groupe 9-fluorénylméthyle, et R³ est un résidu alkyle linéaire ou ramifié ayant de 1 à 4 atomes de carbone, et la réaction s'effectue dans un mélange de solvants composé de R⁴OH, R⁴ étant identique à R³, et de pyridine, diméthylformamide ou acétonitrile.
